# EUROPEAN PATENT SPECIFICATION

(11) **EP 1 515 685 B1**
(45) Date of publication and mention of the grant of the patent: **12.03.2008**
(21) Application number: 03760650.6
(22) Date of filing: 02.06.2003
(51) Int. Cl.: A61K 8/88, A61Q 1/02, A61Q 1/10

(54) **COMPOSITIONS CONTAINING AT LEAST ONE OIL STRUCTURED WITH AT LEAST ONE SILICONE-POLYAMIDE POLYMER, AND AT LEAST ONE FILM-FORMING POLYMER AND METHODS THEREOF**
ZUSAMMENSETZUNGEN, DIE MINDESTENS EIN ÖL, DAS MIT MINDESTENS EINEM SILICON-POLYAMID-POLYMER STRUKTURIERT IST, UND MINDESTENS EIN FILMBILDENDES POLYMER ENTHALTEN, UND VERFAHREN DAFÜR
COMPOSITIONS CONTENANT AU MOINS UNE HUILE STRUCTUREE AVEC AU MOINS UN POLYMERE SILICONE-POLYAMIDE, ET AU MOINS UN POLYMERE FILMOGENE, ET PROCEDES CORRESPONDANT

(30) Priority: 12.06.2002 US 166760
(43) Date of publication of application: 23.03.2005
(73) Proprietor: L'ORÉAL, 75008 Paris (FR)
(72) Inventor: LU, Shaoxiang, Plainsboro, NJ 08536 (US); BLIN, Xavier, F-75015 Paris (FR); MONDET, Jean, F-93600 Aulnay-sous-Bois (FR)
(74) Representative: Boulard, Denis
(86) International application number: PCT/EP2003/006462
(87) International publication number: WO 2004/000247

(56) References cited:
- WO-A-03/013447
- US-A- 5 874 069
- US-A- 5 919 441
- US-A- 5 981 680
- US-A- 6 051 216

## Description

The present invention relates to a care and/or treatment and/or make-up composition for the skin, including the scalp, and/or for the lips of human beings, and/or for keratinous materials, such as keratinous fibers, containing a liquid fatty phase, structured with a specific polymer.

This composition can be stable over time and may be in the form of a stick of make-up such as lipstick, the application of which can produce a glossy deposit with good staying power or long-wearing properties.

It is common to find a structured, i.e., gelled and/or rigidified, liquid fatty phase in cosmetic or dermatological products; this is especially the case in solid compositions such as deodorants, lip balms, lipsticks, concealer products, eye shadows and cast foundations. This structuring may be obtained with the aid of waxes and/or fillers. Unfortunately, these waxes and fillers may have a tendency to make the composition matte, which may not always be desirable, in particular for a lipstick or an eye shadow. Consumers are always on the lookout for a lipstick in stick form which can deposit a film with good staying power or long wearing properties but which is also increasingly glossy.

The structuring of the liquid fatty phase may make it possible in particular to limit its exudation (or syneresis) from solid compositions, particularly in hot and humid areas and, furthermore, after deposition on the skin or the lips, to limit the migration of this phase into wrinkles and fine lines, a characteristic particularly desirable in a lipstick or eye shadow. The reason for this is that considerable migration of the liquid fatty phase, particularly when it is charged with coloring agents, may lead to an unpleasant appearance around the lips and the eyes, making wrinkles and fine lines particularly prominent. Consumers often state this migration as being a major drawback of conventional lipsticks and eye shadows. The term "migration" means movement of the composition beyond its initial site of application.

Gloss of a lipstick or other cosmetic is generally associated with the nature of the liquid fatty phase. Thus, it may be possible to reduce the amount of waxes and/or fillers in the composition in order to increase the gloss of a lipstick, but in that case the migration of the liquid fatty phase may increase. In other words, the amounts of waxes and of fillers required to prepare a stick of suitable hardness that does not exude at room temperature are a restricting factor on the gloss of the deposit.

To overcome at least one of these drawbacks, it has been envisaged replacing all or some of the waxes and/or fillers with polymers for structuring the liquid fatty phase, of the silicone-polyamide type. Unfortunately, the sticks obtained are not mechanically or thermally stable.

Furthermore, make-up compositions should have good staying power or long-wearing properties over time, i.e., little turning of or change in color over time or a gradual or homogeneous change of the deposit over time. The turning of or change in color of the deposit may be due, for lipsticks, to an interaction with saliva and, for foundations and eye shadows, to an interaction with the sweat and sebum secreted by the skin.

Furthermore, the majority of make-up or care compositions, when they are applied to the skin, eyelashes or lips, exhibit the disadvantage of transferring, that is to say of being at least partly deposited and leaving traces on certain substrates with which they may be brought into contact, in particular a glass, a cup, a cigarette, an item of clothing or the skin. This results in mediocre persistence of the applied film, requiring the regular renewal of the application of the composition, in particular a foundation or lipstick composition. In point of fact, it is the wish of users today to beautify their faces, including the lips, and their bodies while spending the least possible time doing so. Furthermore, the appearance of these unacceptable traces, in particular on blouse collars, can dissuade some women from using this type of make up.

The need thus remains for a composition which does not have at least one of the above drawbacks, which has good stability over time, even in hot atmosphere, and which produces a deposit on the skin or the lips that shows good staying power or long-wearing over time and has a glossy appearance. Furthermore, this composition can be easy to manufacture and can give the deposit a sensation of not drying out, both during application and over time.

One subject of the invention is a care and/or make-up and/or treatment composition for the skin and/or the lips of the face and/or for superficial body growths, i.e., keratinous materials, such as nails or keratinous fibers, which makes it possible to overcome at least one of the drawbacks mentioned above.

The inventors have found, surprisingly, that the use of at least one specific structuring polymer combined with at least one film-forming agent makes it possible to obtain a stick whose application to the lips produces a deposit, which can have noteworthy cosmetic properties. In particular, the deposit has good staying power or long-wearing properties over time, and does not transfer on the support applied on the deposit.

Furthermore, the composition can be stable over time at room temperature (25°C) as well as high temperature (typically 47°C). The term "stable" refers to a composition, in particular a stick that is hard and does not collapse over time at room temperature (25°C) and at 47°C for at least 1 month.

The invention applies not only to make-up products for the lips, such as lipsticks, lip glosses and lip pencils, but also to, make-up products for the skin, both of the human face and body, such as foundations optionally cast in stick or dish form, concealer products, blushers, eyeshadows, face powders, transfer tattoos, and make-up products for the eyes such as eyeliners, eye pencils and mascaras, e.g., in cake form, as well as make-up and care products for superficial body growths, for instance keratinous fibers such as the hair, the eyelashes, and the eyebrows or nails.

Another aspect of the invention is a composition comprising at least one liquid fatty phase comprising (i) at least one oil structured with at least one structuring silicone polyamide polymer consisting of a polymer (homopolymer or copolymer) with a weight-average molecular mass ranging from 500 to 500 000, containing at least one moiety of formule (III) 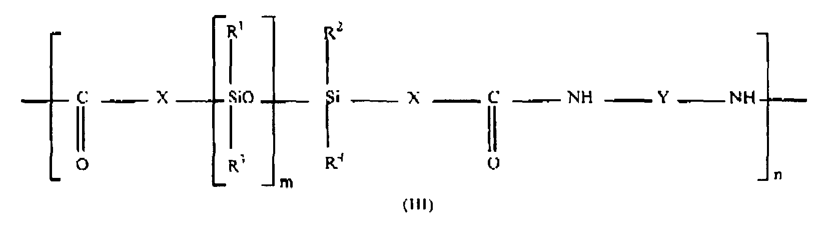 in which:
1) R¹, R², R³ and R⁴, which may be identical or different, represent a group chosen from:
   - linear, branched or cyclic, saturated or unsaturated, C₁ to C₄₀ hydrocarbon-based groups, possibly containing in their chain one or more oxygen, sulphur and/or nitrogen atoms, and possibly being partially or totally substituted with fluorine atoms,
   - C₆ to C₁₀ aryl groups, optionally substituted with one or more C₁ to C₄ alkyl groups,
   - polyorganosiloxane chains possibly containing one or more oxygen, sulphur and/or nitrogen atoms;
2) the groups X, which may be identical or different, represent a linear or branched C₁ to C₃₀ alkylenediyl group, possibly containing in its chain one or more oxygen and/or nitrogen atoms;
3) Y is a saturated or unsaturated, C₁ to C₅₀ linear or branched divalent alkylene, arylene, cycloalkylene, alkylarylene or arylalkylene group, possibly comprising one or more oxygen, sulphur and/or nitrogen atoms, and/or bearing as substituent one of the following atoms or groups of atoms:
   fluorine, hydroxyl, C₃ to C₈ cycloalkyl, C₁ to C₄₀ alkyl, C₅ to C₁₀ aryl, phenyl optionally substituted with 1 to 3 C₁ to C₃ alkyl groups, C₁ to C₃ hydroxyalkyl and C₁ to C₆ aminoalkyl, or
4) Y represents a group corresponding to the formula: 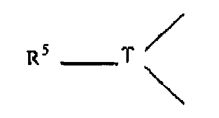 in which
   - T represents a linear or branched, saturated or unsaturated, C₃ to C₂₄ trivalent or tetravalent hydrocarbon-based group optionally substituted with a polyorganosiloxane chain, and possibly containing one or more atoms chosen from O, N and S, or T represents a trivalent atom chosen from N, P and Al, and
   - R⁵ represents a linear or branched C₁ to C₅₀ alkyl group or a polyorganosiloxane chain, possibly comprising one or more ester, amide, urethane, thiocarbamate, urea, thiourea and/or sulphonamide groups, which may possibly be linked to another chain of the polymer;
5) n is an integer ranging from 2 to 500 and preferably from 2 to 200, and m is an integer ranging from 1 to 1 000, preferably from 1 to 700 and better still from 6 to 200, the polymer being solid at room temperature and soluble in said oil at a temperature of from 25 to 250°C, and
   (ii) at least one film-forming agent chosen from the liposoluble acrylic-silicone grafted polymers with a silicone backbone, acrylic grafts or with an acrylic backbone, silicone grafts,
   said oil having an affinity with said structuring polymer and optionally with the film-forming agent, and
   the liquid fatty phase, the polymer and the film-forming agent forming a physiologically acceptable medium,
   iii) further comprising at least one coloring agent.

In one embodiment, the composition of the invention comprises at least one aqueous phase. In that case, the composition can be in the form of a dispersion of the aqueous phase in the liquid fatty phase or in the form of a dispersion of the liquid fatty phase in the aqueous phase. In one embodiment, the composition of the invention is in the form of an emulsion. In one embodiment, the aqueous phase can contain a compound soluble in water such as a monoalcohol having 2 to 8 carbon atoms, a polyol or acetone.

The film-forming organic polymer is: a liposoluble film-forming polymer.

The polymer structuring the liquid fatty phase is solid at room temperature (25°C) and atmospheric pressure (760 mm Hg) and soluble the oil comprised in the liquid fatty phase at a temperature of from 25 to 250°C.

As used herein, the expression "polymer" means a compound having at least two repeating units, preferably at least three repeating unit, more preferably at least ten repeating unit.

In the composition according to the present invention, the structuring polymer represents 0.5 to 80 % by weight, preferably 2 to 60 % by weight, more preferably 5 to 40 % by weight, of the total weight of the composition.

Moreover, the structuring polymer preferably represents 0.1 to 50 % by weight of the weight of the film-forming polymer together with the oil included in the liquid fatty phase.

The liquid fatty phase preferably contains at least 30%, and better still at least 40% by weight of silicone oil.

The composition of the invention can be in the form of a paste, a solid or a more or less viscous cream. It can be a single or multiple emulsion, such as an oil-in-water or water-in-oil emulsion or an oil-in-water-in-oil emulsion, or a water-in-oil-in-water emulsion, or a rigid or soft gel containing an oily continuous phase. For example, the liquid fatty phase can be the continuous phase of the composition. In one embodiment, the composition is anhydrous. In one embodiment, the composition is in a form cast as a stick or in a dish, for example solid, and further example, in the form of an oily rigid gel, such as an anhydrous gel, e.g., an anhydrous stick. In a further embodiment, the composition is in the form of an opaque or translucent rigid gel (depending on the presence or absence of pigments), and in a specific example, the liquid fatty phase forms the continuous phase. In one embodiment, the composition is chosen from molded and poured sticks.

The structuring of the liquid fatty phase can be modified depending on the nature of the structuring polymer that is used, and may be such that a rigid structure in the form of a stick is obtained. When these sticks are colored, they make it possible, after application, to obtain a uniformly colored and glossy deposit, which does not migrate, and/or which has good staying power, in particular of the color over time, and/or which does not transfer.

The composition of the invention can be a composition for the lips, such as a lipstick composition in stick form or a composition for the skin, such as a foundation. *Structuring polymer of the polyorganosiloxane type*

The polymers used as structuring agents in the composition of the invention are polymers of the polyorganosiloxane type such as those described in documents US-A-5 874 069, US-A-5 919 441, US-A-6 051 216 US-A-5 981 680 and US 6 051216. Nevertheless, these documents specifically deal with deodorant and antiperspirant compositions.

The structuring polymer is a polymer comprising at least one moiety of formula (III): 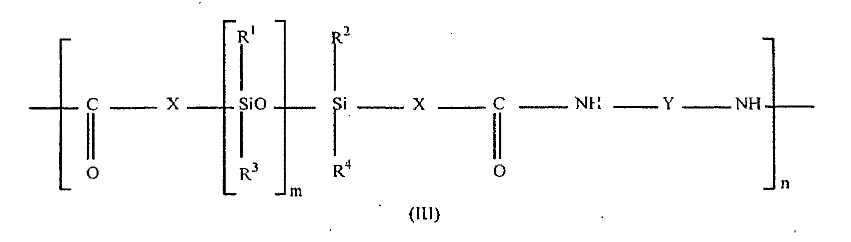 in which R¹, R², R³, R⁴, X, Y, m and n are as defined above.

Such a moiety may be obtained:
- either by a condensation reaction between a silicone containing α,ω-carboxylic acid ends and one or more diamines, according to the following reaction scheme: 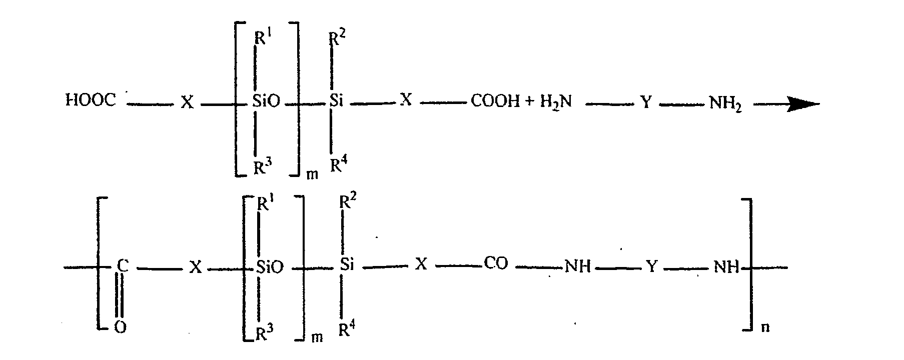
- or by reaction of two molecules of α-unsaturated carboxylic acid with a diamine according to the following reaction scheme: 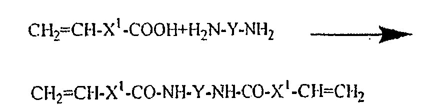 followed by the addition of a siloxane to the ethylenic unsaturations, according to the following scheme:

   CH₂=CH-X¹-CO-NH-Y-NH-CO-X¹-CH=CH₂ 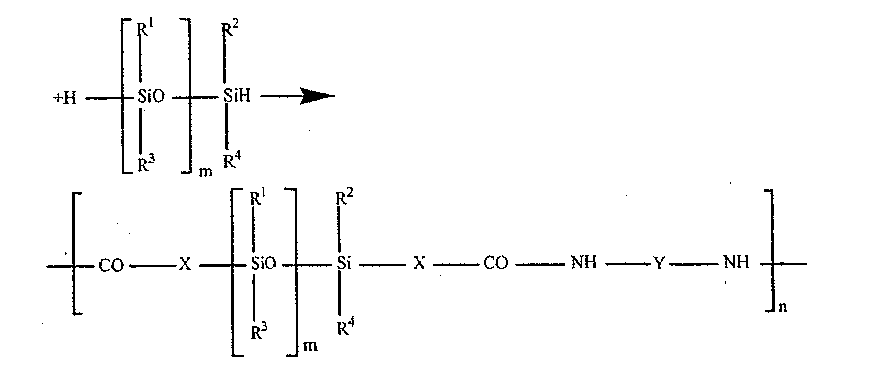
in which X¹-(CH₂)₂- corresponds to X defined above and Y, R¹ R², R³, R⁴ and m are as defined above; In these polyamides of formula (III), m is preferably in the range from 1 to 700, more preferably from 15 to 500 and better still from 15 to 45, and n is in particular in the range from 1 to 500, preferably from 1 to 100 and better still from 4 to 25,
- X is preferably a linear or branched alkylene chain containing from 1 to 30 carbon atoms and in particular 3 to 10 carbon atoms, and
- Y is preferably an alkylene chain that is linear or branched or that possibly comprises rings and/or unsaturations, containing from 1 to 40 carbon atoms, in particular from 1 to 20 carbon atoms and better still from 2 to 6 carbon atoms, in particular 6 carbon atoms.

In formula (III), the alkylene group representing X or Y can optionally contain in its alkylene portion at least one of the following elements:
1°) 1 to 5 amide, urea or carbamate groups,
2°) a C₅ or C₆ cycloalkyl group, and
3°) a phenylene group optionally substituted with 1 to 3 identical or different C₁ to C₃ alkyl groups.

In formulae (III) and (IV), the alkylene groups may also be substituted with at least one element chosen from the group consisting of:
- a hydroxyl group,
- a C₃ to C₈ cycloalkyl group,
- one to three C₁ to C₄₀ alkyl groups,
- a phenyl group optionally substituted with one to three C₁ to C₃ alkyl groups,
- a C₁ to C₃ hydroxyalkyl group, and
- a C₁ to C₆ aminoalkyl group.

In these formulae (III) and (IV), Y may also represent: 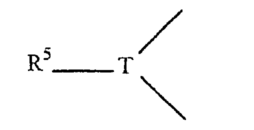 in which R⁵ represents a polyorganosiloxane chain and T represents a group of formula: 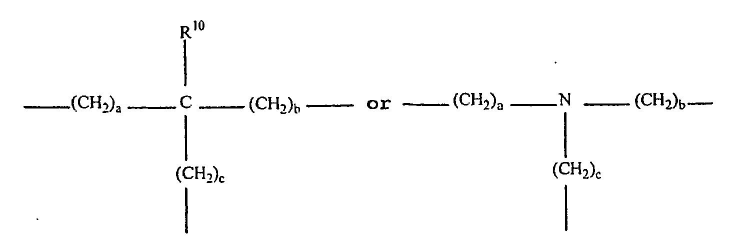 in which a, b and c are, independently, integers ranging from 1 to 10, and R¹⁰ is a hydrogen atom or a group such as those defined for R¹, R², R³ and R⁴.

In formulae (III), R¹, R², R³ and R⁴ preferably represent, independently, a linear or branched C₁ to C₄₀ alkyl group, preferably a CH₃, C₂H₅, n-C₃H₇ or isopropyl group, a polyorganosiloxane chain or a phenyl group optionally substituted with one to three methyl or ethyl groups.

As has been seen previously, the polymer may comprise identical or different moieties of formula (III). Thus, the polymer may be a polyamide containing several moieties of formula (III) of different lengths, i.e. a polyamide corresponding to the formula: 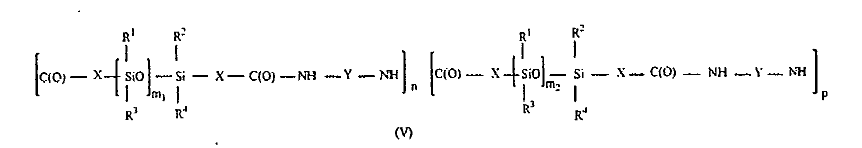 in which X, Y, n and R¹ to R⁴ have the meanings given above, m₁ and m₂, which are different, are chosen in the range from 1 to 1 000, and p is an integer ranging from 2 to 300.
In this formula, the moieties may be structured to form either a block copolymer, or a random copolymer or an alternating copolymer. In this copolymer, the moieties may be not only of different lengths, but also of different chemical structures, for example containing different groups Y. In this case, the copolymer may correspond to the formula: 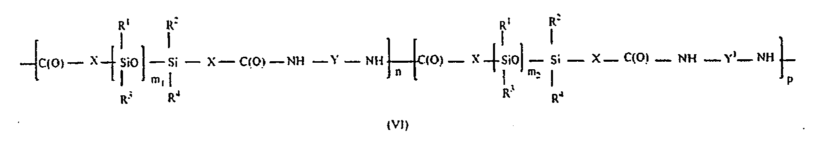 in which R¹ to R⁴, X, Y, m₁, m₂, n and p have the meanings given above and Y¹ is different from Y but chosen from the groups defined for Y. As previously, the various moieties may be structured to form either a block copolymer, or a random copolymer or an alternating copolymer.

According to the invention, the preferred siloxane-based polyamides are:
- polyamides of formula (III) in which m is from 15 to 50;
- mixtures of two or more polyamides in which at least one polyamide has a value of m in the range from 15 to 50 and at least one polyamide has a value of m in the range from 30 to 50;
- mixtures of polyamide of formula (III) combining
   1) 80% to 99% by weight of a polyamide in which n is equal to 2 to 10 and in particular 3 to 6, and
   2) 1 % to 20% of a polyamide in which n is in the range from 5 to 500 and in particular from 6 to 100;
- polyamides of formula (III) synthesized with at least one portion of an activated diacid (diacid chloride, dianhydride or diester) instead of the diacid;
- polyamides of formula (III) in which X represents -(CH₂)₃- or -(CH₂)₁₀; and
- polyamides of formula (III) in which the polyamides end with a monofunctional chain chosen from the group consisting of monofunctional amines, monofunctional acids, monofunctional alcohols, including fatty acids, fatty alcohols and fatty amines, such as, for example, octylamine, octanol, stearic acid and stearyl alcohol.

According to the invention, the end groups of the polymer chain may end with:
- a C₁ to C₅₀ alkyl ester group by introducing a C₁ to C₅₀ monoalcohol during the synthesis,
- a C₁ to C₅₀ alkylamide group by taking as stopping group a monoacid if the silicone is α,ω-diaminated, or a monoamine if the silicone is an α,ω-dicarboxylic acid.

It is possible to use a copolymer of silicone polyamide and of hydrocarbon-based polyamide, i.e. a copolymer comprising moieties of formula (III) and hydrocarbon-based polyamide moieties. In this case, the polyamide-silicone moieties may be arranged at the ends of the hydrocarbon-based polyamide.

Polyamide-based structuring polymers containing silicones may be produced by silylic amidation of polyamides based on fatty acid dimer. This approach involves the reaction, of free acid sites existing on a polyamide as end sites, with organosiloxane-monoamines and/or organosiloxane-diamines (amidation reaction), or alternatively with oligosiloxane alcohols or oligosiloxane diols (esterification reaction). The esterification reaction requires the presence of acid catalysts, as is known in the art. It is desirable for the polyamide containing free acid sites, used for the amidation or esterification reaction, to have a relatively high number of acid end groups (for example polyamides with high acid numbers, for example from 15 to 20).

For the amidation of the free acid sites of the hydrocarbon-based polyamides, siloxane diamines with 1 to 300, more particularly 2 to 50 and better still 2, 6, 9.5, 12, 13.5, 23 or 31 siloxane groups, may be used for the reaction with hydrocarbon-based polyamides based on fatty acid dimers. Siloxane diamines containing 13.5 siloxane groups are preferred, and the best results are obtained with the siloxane diamine containing 13.5 siloxane groups and polyamides containing high numbers of carboxylic acid end groups.

The reactions may be carried out in xylene to extract the water produced from the solution by azeotropic distillation, or at higher temperatures (about 180 to 200°C) without solvent. Typically, the efficacy of the amidation and the reaction rates decrease when the siloxane diamine is longer, that is to say when the number of siloxane groups is higher. Free amine sites may be blocked after the initial amidation reaction of the diaminosiloxanes by reacting them either with a siloxane acid, or with an organic acid such as benzoic acid.

For the esterification of the free acid sites on the polyamides, this may be performed in boiling xylene with about 1 % by weight, relative to the total weight of the reagents, of para-toluenesulphonic acid as catalyst.

These reactions carried out on the carboxylic acid end groups of the polyamide lead to the incorporation of silicone moieties only at the ends of the polymer chain.

It is also possible to prepare a copolymer of polyamide-silicone, using a polyamide containing free amine groups, by amidation reaction with a siloxane containing an acid group.

It is also possible to prepare a structuring polymer based on a copolymer between a hydrocarbon-based polyamide and a silicone polyamide, by transamidation of a polyamide having, for example, an ethylene-diamine constituent, with an oligosiloxane-α,ω-diamine, at high temperature (for example 200 to 300°C), to carry out a transamidation such that the ethylenediamine component of the original polyamide is replaced with the oligosiloxane diamine.

The copolymer of hydrocarbon-based polyamide and of polyamide-silicone may also be a grafted copolymer comprising a hydrocarbon-based polyamide backbone with pendent oligosiloxane groups.

This may be obtained, for example:
- by hydrosilylation of unsaturated bonds in polyamides based on fatty acid dimers;
- by silylation of the amide groups of a polyamide; or
- by silylation of unsaturated polyamides by means of an oxidation, that is to say by oxidizing the unsaturated groups into alcohols or diols, to form hydroxyl groups that are reacted with siloxane carboxylic acids or siloxane alcohols. The olefinic sites of the unsaturated polyamides may also be epoxidized and the epoxy groups may then be reacted with siloxane amines or siloxane alcohols.

As examples of polymers that may be used, mention may be made of the silicone polyamides obtained in accordance with Examples 1 and 2 of document US-A-5 981 680.

The at least one structuring polymer in the compositions of the invention may have a softening point greater than 50°C, such as from 65°C to 190°C, and for example less than 150°C, and further such as from 70°C to 130°C, and even further such as from 80°C to 105°C. This softening point may be lower than that of structuring polymers used in the art which may facilitate the use of the at least one structuring polymer of the present invention and may limit the degradation of the liquid fatty phase. These polymers may be non-waxy polymers.

The softening point can be measured by a well-known method as "Differential Scanning Calorimetry" (i.e. DSC method) with a temperature rise of 5 to 10°C/min. They have good solubility in the silicone oils and produce macroscopically homogeneous compositions. Preferably, they have an average molecular mass from 500 to 200,000, for example from 1,000 to 100,000 and preferably from 2,000 to 30,000.

The structuring polymer is a silicone polyamide, also called a polyamide-modified silicone.

Another embodiment of the invention relates to a mascara, an eyeliner, a foundation; a lipstick, a blusher, a make-up product for the body, an eyeshadow, a face powder, a concealer product, comprising a composition comprising at least one liquid fatty phase in the mascara, eyeliner, foundation, lipstick, blusher, make-up product for the body, eyeshadow, face powder, concealer product, which comprises a composition according to claim 1.

Another embodiment of the invention relates to a method for care, make-up or treatment of keratin materials comprising applying to the keratin materials a composition according to claim 1.

Depending on the intended application, such as a stick, hardness of the composition may also be considered. The hardness of a composition may, for example, be expressed in gramforce (gf). The composition of the present invention may, for example, have a hardness ranging from 20 gf to 2000 gf, such as from 20 gf to 900 gf, and further such as from 20 gf to 600 gf.

This hardness is measured in one of two ways. A first test for hardness is according to a method of penetrating a probe into the composition and in particular using a texture analyzer (for example TA-XT2i from Rheo) equipped with an ebonite cylinder of height 25 mm and diameter 8 mm. The hardness measurement is carried out at 20°C at the center of 5 samples of the composition. The cylinder is introduced into each sample of composition at a pre-speed of 2 mm/s and then at a speed of 0.5 mm/s and finally at a post-speed of 2 mm/s, the total displacement being 1 mm. The recorded hardness value is that of the maximum peak observed. The measurement error is ± 50gf.

The second test for hardness is the "cheese wire" method, which involves cutting an 8.1 mm or preferably 12.7 mm in diameter stick composition and measuring its hardness at 20°C using a DFGHS 2 tensile testing machine from Indelco-Chatillon Co. at a speed of 100 mm/minute. The hardness value from this method is expressed in grams as the shear force required to cut a stick under the above conditions. According to this method, the hardness of compositions according to the present invention which may be in stick form may, for example, range from 30 gf to 300 gf, such as from 30 gf to 250 gf, for a sample of 8.1 mm in diameter stick, and further such as from 30 gf to 200 gf, and also further such as from 30 gf to 120 gf for a sample of 12.7 mm in diameter stick.

The hardness of the composition of the present invention may be such that the compositions are self-supporting and can easily disintegrate to form a satisfactory deposit on a keratinous material. In addition, this hardness may impart good impact strength to the inventive compositions, which may be molded or cast, for example, in stick or dish form.

The skilled artisan may choose to evaluate a composition using at least one of the tests for hardness outlined above based on the application envisaged and the hardness desired. If one obtains an acceptable hardness value, in view of the intended application, from at least one of these hardness tests, the composition falls within the scope of the invention.

As is evident, the hardness of the composition according to the invention may, for example, be such that the composition is advantageously self-supporting and can disintegrate easily to form a satisfactory deposit on the skin and/or the lips and/or superficial body growths, such as keratinous fibers. In addition, with this hardness, the composition of the invention may have good impact strength.

According to the invention, the composition in stick form may have the behavior of a deformable, flexible elastic solid, giving noteworthy elastic softness on application. The compositions in stick form of the prior art do not have these properties of elasticity and flexibility.

### Liquid Fatty Phase

For the purposes of the invention, the expression "liquid fatty phase" means a fatty phase which is liquid at room temperature (25°C) and atmospheric pressure (760 mmHg, i.e. 101 KPa), composed of one or more fatty substances that are liquid at room temperature, also referred to as oils, that are generally mutually compatible, i.e. forming a homogeneous phase macroscopically. The expression "liquid fatty substance" means a non-aqueous liquid medium which is immiscible in all proportions with water, for example, a hydrocarbon-based compound comprising one or more carbon chains each containing at least 5 carbon atoms and possibly comprising at least one polar group chosen from carboxylic acid, hydroxyl, polyol, amine, amide, phosphoric acid, phosphate, ester, ether, urea, carbamate, thiol, thioether and thioester, a silicone compound optionally comprising carbon chains at the end or pendant, these chains optionally being substituted with a group chosen from fluoro, perfluoro, (poly)amino acid, ether, hydroxyl, amine, acid and ester groups; or a fluoro or perfluoro compound such as fluorohydrocarbons or perfluorohydrocarbons containing at least 5 carbon atoms, possibly comprising a hetero atom chosen from N, O, S and P and optionally at least one function chosen from ether, ester, amine, acid, carbamate, urea, thiol and hydroxyl groups.

The at least one liquid fatty phase, in one embodiment, may comprise at least one oil having an affinity with the structuring polymer and optionally with the film-forming polymer. The at least one oil, for example, may be chosen from polar oils and apolar oils including hydrocarbon-based liquid oils and oily liquids at room temperature. In one embodiment, the composition of the invention comprises at least one structuring polymer, at least one film-forming agent and at least one apolar oil. The polar oils of the invention, for example, may be added to an apolar oil, the apolar oils acting in particular as co-solvent for the polar oils.

The liquid fatty phase of the composition may contain more than 30%, for example, more than 40%, of liquid oil(s) containing a group similar to that of the units of the structuring polymer, and for example from 50% to 100%. In one embodiment, the liquid fatty phase structured with a silicone-polyamide-type skeleton contains a high quantity, *i.e.,* greater than 30%, for example greater than 40% relative to the total weight of the liquid fatty phase, or from 50% to 100%, of at least one apolar, such as hydrocarbon-based oil, silicone oils or mixtures thereof. For the purposes of the invention, the expression "hydrocarbon-based oil" means an oil essentially comprising carbon and hydrogen atoms, optionally with at least one group chosen from hydroxyl, ester, carboxyl and ether groups. With such a fatty phase, the at least one film-forming agent may, for example, contain an amine, amide, urethane or silicone group.

For a liquid fatty phase structured with a polymer containing a partially silicone-based skeleton, this fatty phase may contain more than 30%, for example, more than 40%, relative to the total weight of the liquid fatty phase and, for example, from 50% to 100%, of at least one silicone-based liquid oil, relative to the total weight of the liquid fatty phase. In this embodiment, the at least one film-forming agent may comprise a silicone group.

For example, the at least one polar oil useful in the invention may be chosen from:
- hydrocarbon-based plant oils with a high content of triglycerides comprising fatty acid esters of glycerol in which the fatty acids may have varied chain lengths from C₄ to C₂₄, these chains possibly being chosen from linear and branched, and saturated and unsaturated chains; these oils can be chosen from, for example, wheat germ oil, corn oil, sunflower oil, karite butter, castor oil, sweet almond oil, macadamia oil, apricot oil, soybean oil, cotton oil, alfalfa oil, poppy oil, pumpkin oil, sesame oil, marrow oil, rapeseed oil, avocado oil, hazelnut oil, grape seed oil, blackcurrant seed oil, evening primrose oil, millet oil, barley oil, quinoa oil, olive oil, rye oil, safflower oil, candlenut oil, passion flower oil and musk rose oil; or alternatively caprylic/capric acid triglycerides such as those sold by Stearineries Dubois or those sold under the names Miglyol 810, 812 and 818 by Dynamit Nobel;
- synthetic oils or esters of formula R₅COOR₆ in which R₅ is chosen from linear and branched fatty acid residues containing from 1 to 40 carbon atoms and R₆ is chosen from, for example, a hydrocarbon-based chain containing from 1 to 40 carbon atoms, on condition that R₅ + R₆ ≥ 10, such as, for example, purcellin oil (cetostearyl octanoate), isononyl isononanoate, C₁₂-C₁₅ alkyl benzoates, isopropyl myristate, 2-ethylhexyl palmitate, isostearyl isostearate and alkyl or polyalkyl octanoates, decanoates or ricinoleates; hydroxylated esters such as isostearyl lactate and diisostearyl malate; and pentaerythritol esters;
- synthetic ethers containing from 10 to 40 carbon atoms;
- C₈ to C₂₆ fatty alcohols such as oleyl alcohol; and
- C₈ to C₂₆ fatty acids such as oleic acid, linolenic acid or linoleic acid.

The at least one apolar oil according to the invention is chosen from, for example, silicone oils chosen from volatile and non-volatile, linear and cyclic polydimethylsiloxanes (PDMSs) that are liquid at room temperature; polydimethylsiloxanes comprising alkyl or alkoxy groups which are pendant and/or at the end of the silicone chain, the groups each containing from 2 to 24 carbon atoms; phenylsilicones such as phenyl trimethicones, phenyl dimethicones, phenyl trimethylsiloxy diphenylsiloxanes, diphenyl dimethicones, diphenyl methyldiphenyl trisiloxanes and 2-phenylethyl trimethylsiloxysilicates; hydrocarbons chosen from linear and branched, volatile and non-volatile hydrocarbons of synthetic and mineral origin, such as volatile liquid paraffins (such as isoparaffins and isododecane) or non-volatile liquid paraffins and derivatives thereof, liquid petrolatum, liquid lanolin, polydecenes, hydrogenated polyisobutene such as Parleam®, and squalane; and mixtures thereof. The structured oils, for example those structured with silicone polyamides may be, in one embodiment, apolar oils, such as silicone oils.

In one embodiment, the liquid fatty phase comprises one or more silicone oils, in particular at least one non-volatile oil chosen from phenylsilicones such as phenyl trimethicones.

In another embodiment, the viscosity of the oil according to the invention, in particular silicone oil, is less than 1000 cSt, and for example less than 100 cSt.

In another embodiment, the liquid fatty phase comprises one or more volatile oils chosen from silicone oils. In one embodiment, the volatile silicone oil is chosen from polydimethylsiloxanes, linear or cyclic, having 2 to 7 silicium atoms and optionally having an alkyl group or an alkoxy group having 2 to 10 carbon atoms.

For the purposes of the invention, the expression "volatile solvent or oil" means any non-aqueous medium capable of evaporating on contact with the skin or the lips in less than one hour at room temperature and atmospheric pressure. The volatile solvent(s) of the invention is(are) organic solvents, such as volatile cosmetic oils that are liquid at room temperature, having a non-zero vapor pressure, at room temperature and atmospheric pressure, ranging in particular from 10⁻² to 300 mmHg (1.33 to 40 000 Pa) and, for example, greater than 0.03 mmHg (4Pa) and further example greater than 0.3 mmHg (40 Pa). The expression "non-volatile oil" means an oil which remains on the skin or the lips at room temperature and atmospheric pressure for at least several hours, such as those having a vapor pressure of less than 10⁻² mmHg (1.33 Pa).

According to the invention, these volatile solvents may facilitate the staying power or long wearing properties of the composition on the skin, the lips or superficial body growths such as nails and keratinous fibers. The solvents can be chosen from hydrocarbon-based solvents, silicone solvents optionally comprising alkyl or alkoxy groups that are pendant or at the end of a silicone chain, and a mixture of these solvents.

The volatile oil(s), in one embodiment, can be present in an amount ranging from 0% to 95.5% relative to the total weight of the composition, such as from 2% to 75% or, for example, from 10% to 45%. This amount will be adapted by a person skilled in the art according to the desired staying power or long wearing properties.

In practice, the total liquid fatty phase can be, for example, present in an amount ranging from 1 % to 99% by weight relative to the total weight of the composition, for example from 5 % to 99 %, 5% to 95.5 %, from 10 % to 80 % or from 20 % to 75 %.

The at least one liquid fatty phase of the composition of the invention may further comprises a dispersion of lipid vesicles. The composition of the invention may also, for example, be in the form of a fluid anhydrous gel, a rigid anhydrous gel, a fluid simple emulsion, a fluid multiple emulsion, a rigid simple emulsion or a rigid multiple emulsion. The simple emulsion or multiple emulsion may comprise a continuous phase chosen from an aqueous phase optionally containing dispersed lipid vesicles, or a fatty phase optionally containing dispersed lipid vesicles. In one embodiment, the composition has a continuous oily phase or fatty phase and is more specifically an anhydrous composition in, for example, a stick or dish form. An anhydrous composition is one that has less than 10% water by weight, such as, for example, less than 5% by weight. *Film-forming agent*

The composition of the invention also contains at least one liposoluble film-forming agent.

The liposoluble film-forming polymers are:
the grafted acrylic-silicone polymers with a silicone backbone, acrylic grafts or with an acrylic backbone, silicone grafts such as the product sold under the name SA 70.5 by 3M and described in the patents US 5,725,882, US 5,209,924, US 4,972,037, US 4,981,903, US 4,981,902, US 5,468,477 and in the patents US 5,219,560, EP 0 388 582.

According to a preferred embodiment, the film-forming polymer may be purchased from Minnesota Mining and Manufacturing Company under the trade names "Silicone Plus" polymers. For example, poly(isobutyl methacrylate-co-methyl FOSEA)-g-poly(dimethylsiloxane) is sold under the trade name SA 70-5 IBMMF.

According to another preferred form of this invention, the film-forming polymer is chosen from among the silicone polymers grafted with non-silicone organic monomers. These polymers may be liposoluble, lipodispersible, water-soluble or dispersible in an aqueous medium, as the case may.

Said grafted silicone polymer or polymers, with a polysiloxane backbone grafted with non-silicone organic monomers containing a main silicone (or polysiloxane (≡Si-O-)ₙ) chain on which there is grafted, within said chain as well as possibly on at least one of its ends, at least one organic group not comprising silicone.

The polymers with a polysiloxane backbone grafted with non-silicone organic monomers according to the invention may be existing commercial products or else obtained according to any means known to the person skilled in the art, in particular by reaction between (i) a starting silicone correctly functionalized on one or more of these silicon atoms and (ii) a non-silicone organic compound itself correctly functionalized with a function which is capable of reacting with the functional group or groups borne by said silicone by forming a covalent bond; a classic example of such a reaction is the hydroxsylilation reaction between =Si-H groups and CH₂=CH- vinyl groups, or else the reaction between -SH thio-functional groups with these same vinyl groups.

Examples of polymers with a polysiloxane backbone grafted with non-silicone organic monomers suitable for implementation of this invention, as well as the specific method of preparation thereof, are described in particular in patent applications EP-A-0 582 152, WO 93/23009 and WO 95/03776, the teachings of which are included in full in this description by way of non-restrictive references.

According to a particularly preferred embodiment of this invention, the silicone polymer, with a polysiloxane backbone grafted with non-silicone organic monomers which is utilized is composed of the result of radical copolymerization between, on the one hand, at least one non-silicone anionic organic monomer having an ethylene unsaturation and/or a non-silicone hydrophobic organic monomer having an ethylene unsaturation and, on the other, a silicone having in its chain at least one, and preferably several, functional groups capable of reacting on said ethylene unsaturations of said non-silicone monomers by forming a covalent bond, in particular thio-functional groups.

According to this invention, said anionic monomers with ethylene unsaturation preferably are chosen, alone or as a mixture, from among the linear or branched unsaturated carboxylic acids, possibly neutralized in whole or in part in the form of a salt, this or these unsaturated carboxylic acid(s) being able to be most particularly acrylic acid, methacrylic acid, maleic acid, maleic anhydride, itaconic acid, fumaric acid and crotonic acid. The suitable salts are in particular alkaline, alkaline-earth and ammonium salts. It likewise will be noted that, in the final grafted silicone polymer, the organic group with an anionic nature which is composed of the result of radical (homo)polymerization of at least one unsaturated carboxylic-acid-type anionic monomer may be, after reaction, post-neutralized with a base (soda, ammonia,...) to bring it to the form of a salt.

According to this invention, the hydrophobic monomers with ethylene unsaturation preferably are chosen, alone or as a mixture, from among the alkanol acrylic acid esters and/or the alkanol methacrylic acid esters. The alkanols preferably are C₁-C₁₈ and more particularly C₁-C₁₂. The preferred monomers are chosen from within the group consisting of isooctyl (meth)acrylate, isononyl (meth)acrylate, 2-ethylhexyl (meth)acrylate, lauryl (meth)acrylate, isopentyl (meth)acrylate, n-butyl (meth)acrylate, isobutyl (meth)acrylate, methyl (meth)acrylate, tertio-butyl (meth)acrylate, tridecyl (meth)acrylate, stearyl (meth)acrylate or mixtures thereof.

A family of silicone polymers with a polysiloxane backbone grafted with non-silicone organic monomers particularly well suited for implementation of this invention consists of silicone polymers comprising in their structure the moiety of formula IV below: 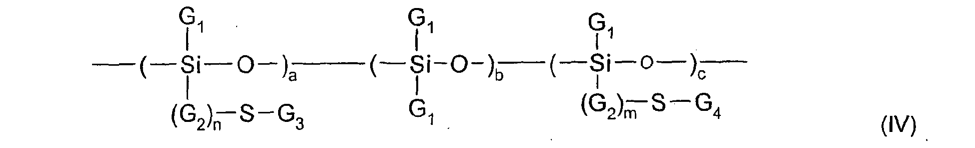 in which the radicals G₁, identical or different, represent hydrogen or a C₁-C₁₀ alkyl radical or even a phenyl radical; the radicals G₂, identical or different, represent represents [sic] a C₁-C₁₀ alkylene group; G₃ represents a polymeric residue resulting from (homo)polymerization of at least one anionic monomer with ethylene unsaturation; G₄ represents a polymeric residue resulting from (homo)polymerization of at least one monomer of at least one hydrophobic monomer [sic] with ethylene unsaturation; m and n are equal to 0 or 1; a is a whole number ranging from 0 to 50; b is a whole number which may range between 10 and 350, c is a whole number ranging from 0 to 50; on condition that one of the parameters a and c is other than 0.

The moiety of formula (IV) hereinabove preferably has at least one, and more preferably still, all of the following characteristics:
- the G₁ radicals denote an alkyl radical, preferably the methyl radical;
- n is not zero, and the G₂ radicals represent a divalent C₁-C₃ radical, preferably a propylene radical;
- G₃ represents a polymeric radical resulting from (homo)polymerization of at least one monomer of the carboxylic acid type with ethylene unsaturation, preferably acrylic acid and/or methacrylic acid;
- G₄ represents a polymeric radical resulting from (homo) polymerization of at least one monomer of the C₁-C₁₀ alkyl (meth)acrylate type, preferably isobutyl or methyl (meth)acrylate.

Examples of silicone polymers corresponding to the formula (IV) are in particular polydimethylsiloxanes (PDMS) on which there are grafted, through a thiopropylene-type secondary linking, mixed polymer moieties of the poly(meth)acrylic acid type and of the alkyl poly(meth)acrylate type.

Other examples of silicone polymers corresponding to formula (IV) are in particular polydimethylsiloxanes (PDMS) on which there are grafted, through a thiopropylene-type secondary linking, polymer moieties of the isobutyl poly(meth)acrylate type.

Such polymers include polymers comprising at least one group of the formula: 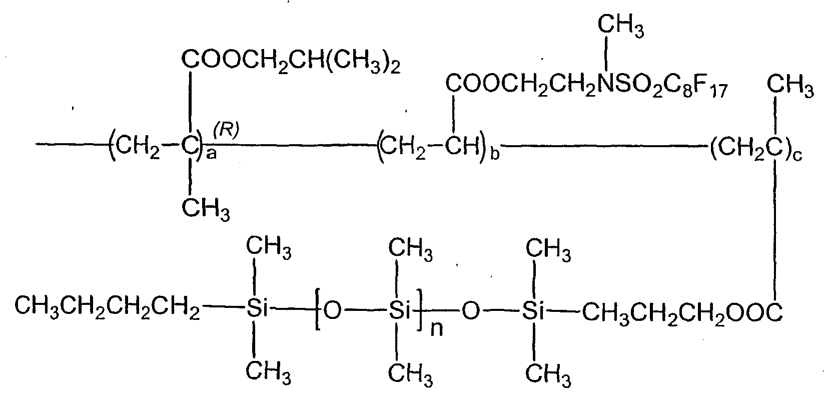 wherein
*a, b,* and *c*, which may be identical or different, are each a number ranging from 1 to 100,000; and the terminal groups, which may be identical or different, are each chosen from C₁-C₂₀ linear alkyl groups, C₃-C₂₀ branched chain alkyl groups, C₃-C₂₀ aryl groups, C₁-C₂₀ linear alkoxy groups, and C₃-C₂₀ branched alkoxy groups.

Such polymers are disclosed in U.S. Patent Nos. 4,972,037, 5,061,481, 5,209,924, 5,849,275, and 6,033,650, and WO 93/23446 and WO 95/06078.

Another family of silicone polymers with a polysiloxane backbone grafted with non-silicone organic monomers particularly well suited to implementation of this invention consists of the silicone polymers comprising in their structure the moiety of formula (V) below 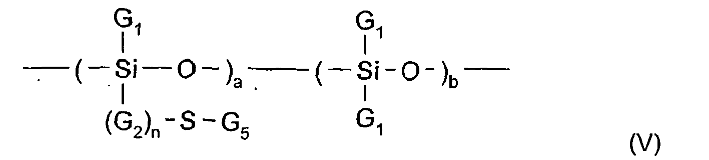 in which the radicals G₁ and G₂ have the same meaning as before; G₅ represents a polymeric residue resulting from (homo)polymerization of at least one monomer of at least one hydrophobic monomer [sic] with ethylene unsaturation or copolymerization of at least one anionic monomer with ethylene unsaturation and at least one hydrophobic monomer with ethylene unsaturation; n is equal to 0 or 1; a is a whole number ranging from 0 to 50; b is a whole number which may range between 10 and 350; on condition that a is other than 0.

The moiety of formula (V) hereinabove preferably has at least one, and more preferably still all, of the following characteristics:
- the radicals G₁ denote an alkyl radical, preferably the methyl radical;
- n is not zero, and the radicals G₂ represent a C₁-C₃ divalent radical, preferably a propylene radical.

The molecular mass by number of the silicone polymers with a polysiloxane backbone grafted with non-silicone organic monomers of the invention preferably varies from approximately 10,000 to 1,000,000, and more preferably still from approximately 10,000 to 100,000.

The composition may contain from 2-60% by weight, better from 5 to 60%, preferably from 2-30% by weight of film-forming-polymer dry matter. More generally, the total quantity of polymer should be in sufficient quantity to form on the skin and/or the lips a cohesive film which is able to follow the movements of the skin and/or the lips without peeling away or cracking.

When the polymer has a glass transition temperature that is too high for the desired use, a plasticizer may be combined therewith so as to lower this temperature of the mixture used. The plasticizer may be chosen from the plasticizers usually used in the field of application, and especially from compounds which may be solvents for the polymer.

The composition also may contain at least one hydrophilic or hydrophobic plasticizing agent, chosen for its compatibility with the polymer or polymers and in a quantity such that it does not impair the sensitivity of the film to water. Said plasticizing agent may be chosen from among all the compounds known to the person skilled in the art as being capable of fulfilling the sought function. This agent may be water soluble or insoluble in water and possibly may exist in the form of an aqueous dispersion.

There may be cited in particular, alone or as a mixture, the usual plasticizers such as:
- glycols and derivatives thereof such as diethylene glycol ethylether, diethylene glycol methylether, diethylene glycol butylether or even diethylene glycol hexylether, ethylene glycol ethylether, ethylene glycol butylether, ethylene glycol hexylether,
- esters of glycerol,
- the derivatives of propylene glycol and in particular propylene glycol phenylether, propylene glycol diacetate, dipropylene glycol butylether, tripropylene glycol butylether, propylene glycol methylether, dipropylene glycol ethylether, tripropylene glycol methylether and diethylene glycol methylether, propylene glycol butylether,
- esters of acids, in particular carboxylic, such as citrates, phthalates, adipates, carbonates, tartrates, phosphates, sebaceates,
- oxyethylene derivatives such as oxyethylene oils, in particular vegetable oils such as castor oil; silicone oils.

The quantity of plasticizing agent may be chosen by the person skilled in the art on the basis of his general knowledge, in such manner as to obtain a film having the desired mechanical properties, while preserving the composition of the cosmetically acceptable properties.

According to the invention, the system comprising the film-forming polymer or polymers, the possible coalescent agents and the possible plasticizers shall be called "polymeric system"; this polymeric system is to be capable of forming a film on the support on which it is deposited, supple, flexible, cohesive, following the movements of the support (lips or skin) on which it is deposited.

When the composition is in the form of a foundation or a lipstick, the film obtained with said composition preferably shall bear out, in the measurement conditions defined preceding the examples, at least one of the following physico-chemical conditions:
- A Young modulus less than approximately 200 MPa, preferably less than approximately 100 MPa, and preferred less than 80 MPa, and/or
- an elongation in excess of approximately 200% and, preferably, in excess of 300%, and/or
- a hardness less than 110, preferably less than 70, more preferably less than 55.

### Additional Additives

The composition of the invention can also comprise any additive usually used in the field under consideration, chosen in particular from dispersants such as poly(2-hydroxystearic acid), antioxidants, essential oils, preserving agents, fragrances, waxes, fillers, neutralizing agents, cosmetic and dermatological active agents such as, for example, emollients, moisturizers, vitamins, essential fatty acids, sunscreens, and mixtures thereof. These additives may be present in the composition in a proportion of from 0% to 20% (such as from 0.01 % to 20%) relative to the total weight of the composition and further such as from 0.01% to 10% (if present).

The composition of the invention can also contain, as an additive, an aqueous phase containing water that is optionally thickened or gelled with an aqueous-phase thickener or gelling agent and/or containing ingredients soluble in water. The water can represents from 0.01 to 50 %, for example from 0.5 to 30 % relative to the total weight of the composition.

Needless to say, a person skilled in the art will take care to select the optional additional additives and/or the amount thereof such that the advantageous properties of the composition according to the invention are not, or are not substantially, adversely affected by the envisaged addition.

The composition of the invention is in the form of a colored make-up product for the skin, in particular a foundation, optionally having care or treating properties, a blusher, a face powder, an eye shadow, a concealer product, an eyeliner, a make-up product for the body; a make-up product for the lips such as a lipstick, optionally having care or treating properties; a make-up product for superficial body growths such as the nails or the eyelashes, in particular in the form of a mascara cake, or for the eyebrows and the hair, in particular in the form of a pencil.

Needless to say, the composition of the invention should be cosmetically or dermatologically acceptable, i.e. it should contain a non-toxic physiologically acceptable medium and should be able to be applied to the skin, superficial body growths or the lips of human beings. For the purposes of the invention, the expression "cosmetically acceptable" means a composition of pleasant appearance, odor, feel and taste.

The composition advantageously contains at least one cosmetic active agent and/or at least one dermatological active agent, i.e., an agent having a beneficial effect on the skin, lips or body growths. The composition contains at least one coloring agent.

### Coloring agents

The coloring agent according to the invention may be chosen from the lipophilic dyes, hydrophilic dyes, pigments and nacreous pigments (i.e., nacres) usually used in cosmetic or dermatological compositions, and mixtures thereof. This coloring agent is generally present in a proportion of from 0.01 % to 50% relative to the total weight of the composition, such as from 0.5% to 40% and further such as from 5% to 30%, if it is present. In the case of a composition in the form of a free or compacted powder, the amount of coloring agent in the form of solid particles that are insoluble in the medium (nacres and/or pigments) may be up to 90% relative to the total weight of the composition.

The liposoluble dyes are, for example, Sudan Red, D&C Red 17, D&C Green 6, β-carotene, soybean oil, Sudan Brown, D&C Yellow 11, D&C Violet 2, D&C Orange 5, quinoline yellow or annatto. They can represent from 0.1 % to 20% of the weight of the composition, for example, from 0.1% to 6% (if present). The water-soluble dyes are, for example, beetroot juice or methylene blue, and can represent up to 6% of the total weight of the composition.

The pigments may be white or colored, goniochromatic or not, mineral and/or organic, and coated or uncoated. Among the mineral pigments which may be mentioned are titanium dioxide, optionally surface-treated, zirconium oxide, zinc oxide or cerium oxide, as well as iron oxide, chromium oxide, manganese violet, ultramarine blue, chromium hydrate and ferric blue. Among the organic pigments that may be mentioned are carbon black, pigments of D & C type, and lakes based on cochineal carmine or on barium, strontium, calcium or aluminium. The pigments can represent from 0.1 % to 50%, such as from 0.5% to 40% and further such as from 2% to 30% relative to the total weight of the composition, if they are present.

The nacreous pigments may be chosen from white nacreous pigments such as mica coated with titanium or with bismuth oxychloride, colored nacreous pigments such as titanium mica with iron oxides, titanium mica with, in particular, ferric blue or chromium oxide, titanium mica with an organic pigment of the type mentioned above, as well as nacreous pigments based on bismuth oxychloride. They can represent, for example, from 0.1 % to 20% relative to the total weight of the composition, and further such as from 0.1 % to 15%, if they are present.

In one embodiment, the coloring agent is a pigment (nacreous or not).

In another embodiment, the pigment is treated or not treated, and is preferably hydrophobic.

### Waxes

The composition can optionally contain one or more waxes to improve the structuring in stick form, although this rigid form can be obtained in the absence of wax. For the purposes of the present invention, a wax is a lipophilic fatty compound that is solid at room temperature (25°C) and atmospheric pressure (760 mmHg, i.e. 101 KPa), which undergoes a reversible solid/liquid change of state, having a melting point of greater than 40°C and further such as greater than 55°C and which may be up to 200°C, and having an anisotropic crystal organization in the solid state. The size of the crystals is such that the crystals diffract and/or scatter light, giving the composition a cloudy, more or less opaque appearance. By bringing the wax to its melting point, it is possible to make it miscible with oils and to form a microscopically homogeneous mixture, but on returning the temperature of the mixture to room temperature, recrystallization of the wax in the oils of the mixture is obtained. It is this recrystallization in the mixture which is responsible for the reduction in the gloss of the mixture. Thus, the composition advantageously contains little or no wax, and in particular less than 5% wax.

For the purposes of the invention, the waxes are those generally used in cosmetics and dermatology; they are, for example, of natural origin, for instance beeswax, carnauba wax, candelilla wax, ouricury wax, Japan wax, cork fibre wax, sugar cane wax, paraffin wax, lignite wax, microcrystalline waxes, lanolin wax, montan wax, ozokerites and hydrogenated oils such as hydrogenated jojoba oil as well as waxes of synthetic origin, for instance polyethylene waxes derived from the polymerization of ethylene, waxes obtained by Fischer-Tropsch synthesis, fatty acid esters and glycerides that are solid at 40°C, for example, at above 55°C, silicone waxes such as alkyl- and alkoxy-poly(di)methylsiloxanes and/or poly(di)methyl-siloxane esters that are solid at 40°C, for example, at above 55°C.

According to the invention, the melting point values correspond to the melting peak measured by the "Differential Scanning Calorimetry" method with a temperature rise of 5 or 10°C/min.

The composition according to the invention may be manufactured by the known processes that are generally used in cosmetics or dermatology. It may be manufactured by the process which comprises heating the polymer at least to its softening point, adding the film-forming agent(s), the coloring agent(s) and the additive(s) thereto and then mixing everything together until a clear, transparent solution is obtained. After reducing the temperature, the volatile solvent(s) is(are) then added to the mixture obtained. The homogeneous mixture obtained can then be cast in a suitable mould such as a lipstick mould or directly into the packaging articles (case or dish in particular).

Another aspect of the invention is a lipstick composition in stick form at least one liquid fatty phase comprising (i) at least one oil structured with at least one structuring polymer consisting of a polymer (homopolymer or copolymer) with a weight-average molecular mass ranging from 500 to 500 000, containing at least one moiety comprising:
- at least one polyorganosiloxane group, consisting of from 1 to 1 000 organosiloxane units in the chain of the moiety or in the form of a graft, and
- at least two groups capable of establishing hydrogen interactions, chosen from ester, amide, sulphonamide, carbamate, thiocarbamate, urea, thiourea, oxamido, guanamido and biguanidino groups, and combinations thereof, on condition that at least one of the groups is other than an ester group, the polymer being solid at room temperature and soluble in said oil at a temperature of from 25 to 250°C, and
   (ii) at least one film-forming agent dispersed or solubilized in the liquid fatty phase,
   said oil having an affinity with said structuring polymer and optionally with the film-forming agent, and
   the liquid fatty phase, the polymer and the film-forming agent forming a physiologically acceptable medium.

An aspect of the invention is also a care, make-up or treatment cosmetic process for keratin materials of human beings, and in particular the skin, the lips and superficial body growths, comprising the application to the keratin materials of the composition, in particular the cosmetic composition, as defined above.

An aspect of the invention is also a combination (i) of at least one polymer consisting of a polymer (homopolymer or copolymer) with a weight-average molecular mass ranging from 500 to 500 000, containing at least one moiety comprising:
- at least one polyorganosiloxane group, consisting of from 1 to 1 000 organosiloxane units in the chain of the moiety or in the form of a graft, and
- at least two groups capable of establishing hydrogen interactions, chosen from ester, amide, sulphonamide, carbamate, thiocarbamate, urea, thiourea, oxamido, guanamido and biguanidino groups, and combinations thereof, on condition that at least one of the groups is other than an ester group, the polymer being solid at room temperature and soluble in said oil at a temperature of from 25 to 250°C, and
   (ii) at least one film-forming agent,
   in a cosmetic composition or for the manufacture of a physiologically acceptable composition, to obtain a solid composition, such as a wax-free composition, which does not exude and/or which can produce a glossy and/or comfortable deposit on keratin materials, said composition containing an said oil having an affinity with said structuring polymer and optionally with the film-forming agent, and the liquid fatty phase, the polymer and the film-forming agent forming a physiologically acceptable medium.

The compositions of the present invention may also further comprise water, optionally thickened with an aqueous-phase thickener or gelled with a film-forming agent and/or containing ingredients soluble in water.

The invention is illustrated in greater detail in the examples, which follow. The amounts are given as percentages by mass.

### Example 1: Lip Gloss

| Ingredient | Trade Name | %w/w |
|---|---|---|
| Film Former | SA-70 from 3M | 20.0 |
| Polyamidodimethylsiloxane¹ | DC2-8179 | 8.0 |
| Phenyltrimethicone | DC 556 | 65.1 |
| Pigments | | 6.9 |

| | | |
|---|---|---|
| ¹ Dow Corning DC 2-9179 (DP=15) | | |

This composition was supple and elastic.
The film forming polymer is introduced under agitation with a magnetic stirrer after the rest of the formula has been heated. The gloss is introduced into a container and applied using a sponge type applicator.

The composition exhibits better wear when compared with one not containing a film-forming polymer.

### Example 2: Foundation

In phase A, ingredients are mixed well and ground with a Silverson homogenizer at a speed of 6000 rpm.
Separately the phase B1 ingredients are heated to 80 to 85 °C with stirring for 10-15 minutes or until dissolution of the siloxane-polyamide.
Phase A and B1 are then combined in the main beaker and mixed well at 70 to 75 °C.
Phase B2 is added to the main beaker and is mixed until uniform.
Disteardimonium Hectorite is added to the main beaker and dispersed well before adding rest of phase B3 ingredients.
Phase C is heated to 70 to 75 °C in a separate side beaker. Emulsification is carried out by adding phase C to main beaker and homogenizing at medium/high speed.
The batch is cooled to room temperature with a paddle stirrer.

| *PHASE* | *INCI Name* | *%ww* |
|---|---|---|
| | | |
| *A* | Cyclopentasiloxane (and) dimethicone copolyol | 8.0 |
| | Polyglyceryl-4 isostearate (and) hexyl laurate (and) cetyl PEG/PPG-10/1 dimethicone | 3.5 |
| | Treated pigments | 9.9 |
| | | |
| *B1* | Cyclopentasiloxane | 16.1 |
| | Polysiloxane/Polyamide | 1.0 |
| | Silicone-Acrylates | 12.0 |
| | | |
| *B2* | Fillers | 6.0 |
| | | |
| *B3* | Preservative | 0.4 |
| | Disteardimonium Hectorite | 0.6 |
| | Propylene Carbonate | 0.2 |
| | | |
| *C* | Water | Qsp 100 |
| | Magnesium Sulfate | 1.0 |
| | Preservative | 0.7 |
| | Non ionic emulsifier | 0.5 |
| | TOTAL | 100.00 |

### Example 3: Foundation

Is prepared according to the same procedure as described in example 2.

| Phase | Ingredient Name | %w/w |
|---|---|---|
| A | Cyclopentasiloxane and Dimethicone Copolyol | 8.00 |
| | Polyglyceryl-4-isostearate and Hexyl Laurate and Cetyl PEG/PPG-10/1 Dimethicone2 | 3.50 |
| | Treated Pigments | 9.90 |
| | | |
| B1 | Volatile Oil | 26.10 |
| | Siloxane based polyamide | 2.00 |
| | TiO₂/Silicone-Acrylates | 12.00 |
| | | |
| B2 | Fillers | 6.00 |
| | | |
| B3 | Preservative | 0.40 |
| | Disteardimonium Hectorite | 1.00 |
| | Propylene Carbonate | 0.30 |
| | | |
| C | Water | qsp |
| | Magnesium Sulfate | 1.00 |
| | Preservatives | 0.70 |
| | Laureth-4 | 0.50 |
| | | 100.00 |

## Claims

1. Make-up composition comprising at least one liquid fatty phase comprising
(i) at least one oil structured with at least one structuring silicone polyamide polymer consisting of a polymer (homopolymer or copolymer) with a weight-average molecular mass ranging from 500 to 500 000, containing at least one moiety of formule (III) 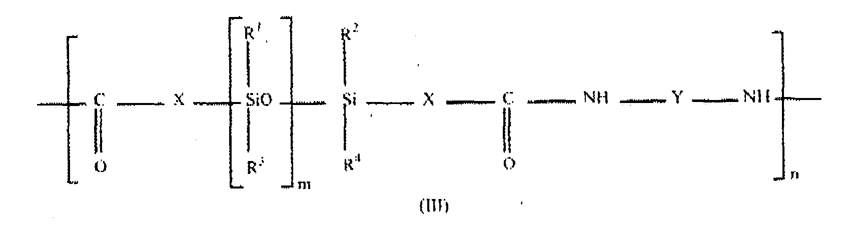 in which:
1) R¹, R², R³ and R⁴, which may be identical or different, represent a group chosen from:
- linear, branched or cyclic, saturated or unsaturated, C₁ to C₁₀ hydrocarbon-based groups, possibly containing in their chain one or more oxygen, sulphur and/or nitrogen atoms, and possibly being partially or totally substituted with fluorine atoms,
- C₆ to C₁₀ aryl groups, optionally substituted with one or more C₁ to C₄ alkyl groups,
- polyorganosiloxane chains possibly containing one or more oxygen, sulphur and/or nitrogen atoms;
2) the groups X, which may be identical or different, represent a linear or branched C₁ to C₃₀ alkylenediyl group, possibly containing in its chain one or more oxygen and/or nitrogen atoms;
3) Y is a saturated or unsaturated, C₁ to C₅₀ linear or branched divalent alkylene, arylene, cycloalkylene, alkylarylene or arylalkylene group, possibly comprising one or more oxygen, sulphur and/or nitrogen atoms, and/or bearing as substituent one of the following atoms or groups of atoms:
fluorine, hydroxyl, C₃ to C₈ cycloalkyl, C₁ to C₁₀ alkyl, C₅ to C₁₀ aryl, phenyl optionally substituted with 1 to 3 C₁ to C₃ alkyl groups, C₁ to C₃ hydroxyalkyl and C₁ to C₆ aminoalkyl, or
4) Y represents a group corresponding to the formula: 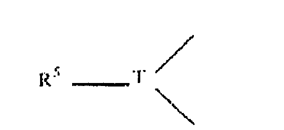 in which
- T represents a linear or branched, saturated or unsaturated, C₃ to C₂₄ trivalent or tetravalent hydrocarbon-based group optionally substituted with a polyorganosiloxane chain, and possibly containing one or more atoms chosen from O, N and S, or T represents a trivalent atom chosen from N, P and Al, and
- R⁵ represents a linear or branched C₁ to C₅₀ alkyl group or a polyorganosiloxane chain, possibly comprising one or more ester, amide, urethane, thiocarbamate, urea, thiourea and/or sulphonamide groups, which may possibly be linked to another chain of the polymer;
5) n is an integer ranging from 2 to 500 and preferably from 2 to 200, and in is an integer ranging from 1 to 1 000. preferably from I to 700 and better still from 6 to 200. the polymer being solid at 25°C and soluble in said oil at a temperature of from 25 to 250°C,
(ii) at least one film-forming polymer chosen from the liposoluble acrylic-silicone grafted polymers with a silicone backbone, acrylic grafts or with an acrylic backbone, silicone grafts,
said oil having an affinity with said structuring polymer and optionally said film-forming polymer, and
the liquid fatty phase, the structuring polymer and the film-forming polymer forming a physiologically acceptable medium,
, and
iii) further comprising at least one coloring agent.

2. Composition according to Claim 1, in which the polymer represents from 0,5% to 80%, preferably from 2% to 60% and better still from 5% to 40%, relative to the total weight of the composition.

3. Composition according to Claim 1, wherein said at least one structuring polymer has a softening point greater than 50°C.

4. Composition according to Claim 1, wherein said at least one structuring polymer has a softening point is less than 150°C.

5. Composition according to Claim 1, wherein said at least one structuring polymer has a softening point ranging from 70°C to 130°C.

6. Composition according to Claim 1, wherein said at least one structuring polymer has a weight-average molecular mass ranging from 500 to 200,000, preferably ranging from 1,000 to 100,000, more preferably ranging from 2,000 to 30,000.

7. Composition according to Claim 1, wherein said composition has a hardness ranging from 30 to 300 gf.

8. Composition according to Claim 1, wherein said composition has a hardness ranging from 30 to 250 gf, preferably from 30 to 200 gf.

9. Composition according to Claim 1, wherein said at least one liquid fatty phase of the composition comprises at least one oil chosen from at least one polar oil and at least one apolar oil having an affinity with the least one structuring polymer.

10. Composition according to claim 1, wherein said at least one polar oil is chosen from:
- hydrocarbon-based plant oils with a high content of triglycerides comprising fatty acid esters of glycerol in which the fatty acids comprise chains having from 4 to 24 carbon atoms, said chains optionally being chosen from linear and branched, and saturated and unsaturated chains;
- synthetic oils or esters of formula R₅COOR₆ in which R₅ is chosen from linear and branched fatty acid residues comprising from 1 to 40 carbon atoms and R₆ is chosen from hydrocarbon-based chain containing form 1 to 40 carbon atoms, with the proviso that R₅ + R₆ ≥ 10;
- synthetic ethers containing from 10 to 40 carbon atoms;
- C₈ to C₂₆ fatty alcohols; and
- C₈ to C₂₆ fatty acids.

11. Composition according to claim 1, wherein said at least one apolar oil is chosen from:
- silicone oils chosen from volatile and non-volatile, linear and cyclic polydimethylsiloxanes that are liquid at room temperature;
- polydimethylsiloxanes comprising alkyl or alkoxy groups which are pendant and/or at the end of the silicone chain, the groups each containing from 2 to 24 carbon atoms;
- phenylsilicones, in particular phenyltrimethicone; and
- hydrocarbons chosen from linear and branched, volatile and non-volatile hydrocarbons of synthetic and mineral origin.

12. Composition according to Claim 1, wherein said at least one liquid fatty phase is present in an amount ranging from 1 % to 99% by weight relative to the total weight of the composition.

13. Composition according to Claim 1, wherein said at least one liquid fatty phase is present in an amount ranging from 10% to 80% by weight relative to the total weight of the composition.

14. Composition according to Claim 1, in which the liquid fatty phase contains more than 30%, preferably more than 40% and better still from 50% to 100% by weight of at least one silicone-based liquid oil.

15. Composition according to Claim 1, wherein said at least one liquid fatty phase comprises at least one volatile solvent chosen from hydrocarbon-based solvents and silicone solvents optionally comprising alkyl or alkoxy groups that are pendant or at the end of a silicone chain.

16. Composition according to Claim 1, wherein the film-forming weight ratio ranges from 2-60%, preferably from 5 to 60 %, more preferably from 2 to 30% by weight of dry compound relative to the total weight of the composition.

17. Composition according to Claim 1, further comprising at least one additional additive chosen from dispersants such as poly(2-hydroxystearic acid), antioxidants, essential oils, preserving agents, fragrances, waxes, fillers, neutralizing agents, cosmetic and dermatological active agents such as, for example, emollients, moisturizers, vitamins, essential fatty acids, sunscreens, and mixtures thereof.

18. Composition according to Claim 1, wherein said at least one coloring agent is chosen from lipophilic dyes, hydrophilic dyes, pigments and nacres.

19. Composition according to claim 1, wherein said at least one coloring agent is present in a proportion of from 0.01% to 50%, preferably from 0.5% to 40% and more preferably from 5% to 30% relative to the total weight of the composition.

20. Composition according to Claim 1, wherein said composition is a solid.

21. Composition according to any one of the preceding claims, wherein said composition is a solid chosen from molded and poured sticks.

22. Composition according to Claim 1, wherein said composition is in the form of a rigid gel.

23. Composition according to Claim 1, wherein said composition is in the form of an anhydrous stick.

24. Composition according to Claim 1, wherein said composition contains an aqueous phase.

25. Composition according to Claim 1. wherein said composition is an emulsion.

26. A mascara, an eyeliner, a foundation, a lipstick, a blusher, a make-up product for the body, an eyeshadow, a face powder, a concealer product, comprising a composition comprising at least one liquid fatty phase in the mascara, eyeliner, foundation, lipstick, blusher, make-up product for the body, eyeshadow, face powder, concealer product, which comprises a composition as described in claim 1.

27. A method for care, make-up or treatment of keratin materials comprising applying to the keratin materials a composition as described in claim 1.

## Patentansprüche

1. Make-up-Zusammensetzung, die mindestens eine flüssige Fettphase umfasst, die umfasst:
(i) mindestens ein Öl, das mit mindestens einem strukturierenden Siliconpolyamidpolymer strukturiert ist, das aus einem Polymer (Homopolymer oder Copolymer) mit einer gewichtsmittleren Molekularmasse, die im Bereich von 500 bis 500000 liegt, besteht, das mindestens eine Einheit der Formel (III) 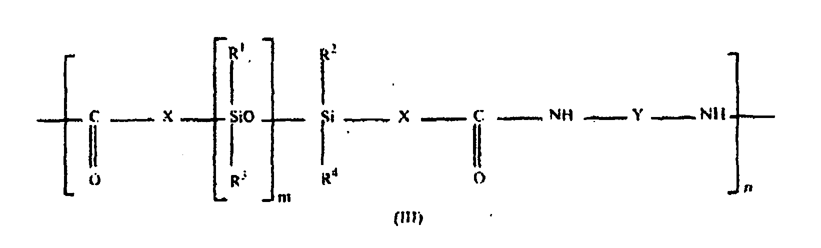 enthält, in der
1) R₁, R₂, R₃ und R₄, die identisch oder verschieden sein können, eine Gruppe darstellen, die ausgewählt wird unter
- Gruppen auf der Basis eines geradkettigen, verzweigten oder cyclischen, gesättigten oder ungesättigten C₁- bis C₄₀-Kohlenwasserstoffs, die in ihrer Kette möglicherweise ein oder mehrere Sauerstoff-, Schwefel- und/ oder Stickstoffatome enthalten und die möglicherweise teilweise oder vollständig mit Fluoratomen substituiert sind,
- C₆- bis C₁₀-Arylgruppen, die optional mit einer oder mehreren C₁- bis C₄-Alkylgruppen substituiert sind,
- Polyorganosiloxanketten, die möglicherweise ein oder mehrere Sauerstoff-, Schwefel- und/oder Stickstoffatome enthalten;
2) die Gruppen X, die identisch oder verschieden sein können, eine geradkettige oder verzweigte C₁- bis C₃₀-Alkylendiylgruppe darstellen, die möglicherweise in ihrer Kette ein oder mehrere Sauerstoff und/oder Stickstoffatome enthält;
3) Y eine gesättigte oder ungesättigte, 1 bis 50 Kohlenstoffatome umfassende, geradkettige oder verzweigte, zweiwertige Alkylen-, Arylen-, Cycloalkylen-, Alkylarylen- oder Arylalkylengruppe ist, die möglicherweise ein oder mehrere Sauerstoff-, Schwefel- und/oder Stickstoffatome umfasst, und/ oder die eines der folgenden Atome oder eine der folgenden Gruppen von Atomen als Substituenten trägt:
Fluor, Hydroxy, C₃- bis C₈-Cycloalkyl, C₁- bis C₄₀-Alkyl,
C₅- bis C₁₀-Aryl, Phenyl, das optional substituiert ist mit 1 bis 3 C₁- bis C₃-Alkylgruppen, C₁- bis C₃-Hydroxyalkyl und C₁- bis C₆-Aminoalkyl, oder
4) Y eine Gruppe darstellt, die der Formel 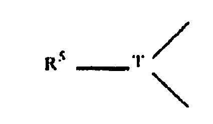 entspricht, in der
- T eine geradkettige oder verzweigte, gesättigte oder ungesättigte, 3 bis 24 Kohlenstoffatome umfassende, dreiwertige oder vierwertige Gruppe auf Kohlenwasserstoffbasis darstellt, die optional mit einer Polyorganosiloxankette substituiert ist und die möglicherweise ein oder mehrere Atome enthält, die unter O, N und S ausgewählt werden, oder in der T ein dreiwertiges Atom darstellt, das unter N, P und Al ausgewählt wird, und
- R₅ eine geradkettige oder verzweigte C₁- bis C₅₀-Alkylgruppe oder eine Polyorganosiloxankette darstellt, die möglicherweise eine oder mehrere Ester-, Amid-, Urethan-, Thiocarbamat-, Harnstoff-, Thioharnstoff- und/oder Sulfonamidgruppen umfasst, die möglicherweise mit einer anderen Kette des Polymers verbunden sein können;
5) n eine ganze Zahl ist, die im Bereich von 2 bis 500 und vorzugsweise 2 bis 200 liegt, und m eine ganze Zahl ist, die im Bereich von 1 bis 1 000, vorzugsweise 1 bis 700 und noch besser 6 bis 200 liegt,
wobei das Polymer bei 25 °C fest ist und in dem Öl bei einer Temperatur im Bereich von 25 bis 250 °C löslich ist,
(ii) mindestens ein filmbildendes Polymer, das unter den fettlöslichen gepfropften Acryl-Silicon-Polymeren mit einem Siliconrückgrat und Acrylpfropfzweigen oder mit einem Acrylrückgrat und Siliconpfropfzweigen ausgewählt wird,
wobei das Öl eine Affinität zu dem strukturierenden Polymer und optional zu dem filmbildenden Polymer hat, und
wobei die flüssige Fettphase, das strukturierende Polymer und das filmbildende Polymer ein physiologisch akzeptables Medium bilden, und
iii) weiterhin mindestens ein Färbemittel.

2. Zusammensetzung nach Anspruch 1, in der das Polymer 0,5 bis 80 %, vorzugsweise 2 bis 60 % und noch besser 5 bis 40 %, bezogen auf das Gesamtgewicht der Zusammensetzung, ausmacht.

3. Zusammensetzung nach Anspruch 1, wobei das mindestens eine strukturierende Polymer einen Erweichungspunkt oberhalb von 50 °C aufweist.

4. Zusammensetzung nach Anspruch 1, wobei das mindestens eine strukturierende Polymer einen Erweichungspunkt unterhalb von 150 °C aufweist.

5. Zusammensetzung nach Anspruch 1, wobei das mindestens eine strukturierende Polymer einen Erweichungspunkt aufweist, der im Bereich von 70 bis 130 °C liegt.

6. Zusammensetzung nach Anspruch 1, wobei das mindestens eine strukturierende Polymer eine gewichtsmittlere Molekularmasse aufweist, die im Bereich von 500 bis 200000, vorzugsweise im Bereich von 1000 bis 100000, noch bevorzugter im Bereich von 2000 bis 30000 liegt.

7. Zusammensetzung nach Anspruch 1, wobei die Zusammensetzung eine Härte aufweist, die im Bereich von 30 bis 300 gf liegt.

8. Zusammensetzung nach Anspruch 1, wobei die Zusammensetzung eine Härte aufweist, die im Bereich von 30 bis 250 gf, vorzugsweise 30 bis 200 gf, liegt.

9. Zusammensetzung nach Anspruch 1, wobei die mindestens eine flüssige Fettphase der Zusammensetzung mindestens ein Öl umfasst, das unter mindestens einem polaren Öl und mindestens einem unpolaren Öl ausgewählt wird und das eine Affinität für das mindestens eine strukturierende Polymer hat.

10. Zusammensetzung nach Anspruch 1, wobei das mindestens eine polare Öl ausgewählt wird unter:
- Pflanzenölen auf Kohlenwasserstoffbasis mit einem hohen Gehalt an Triglyceriden, die Fettsäureester von Glycerin umfassen, in denen die Fettsäuren Ketten aufweisen, die 4 bis 24 Kohlenstoffatome umfassen, wobei die Ketten optional unter geradkettigen und verzweigten und gesättigten und ungesättigten Ketten ausgewählt werden;
- synthetischen Ölen oder Estern der Formel R₅COOR₆, worin R₅ unter geradkettigen und verzweigten Fettsäureresten, die 1 bis 40 Kohlenstoffatome umfassen, ausgewählt wird und worin R₆ unter Ketten auf Kohlenwasserstoffbasis ausgewählt wird, die 1 bis 40 Kohlenstoffatome enthalten, mit der Maßgabe, dass R₅ + R₆ ≥ 10 ist;
- synthetischen Ethern, die 10 bis 40 Kohlenstoffatome enthalten;
- C₈- bis C₂₆-Fettalkoholen; und
- C₈- bis C₂₆-Fettsäuren.

11. Zusammensetzung nach Anspruch 1, wobei das mindestens eine unpolare Öl ausgewählt wird unter:
- Siliconölen, die unter flüchtigen und nichtflüchtigen, geradkettigen und cyclischen Polydimethylsiloxanen, die bei Raumtemperatur flüssig sind;
- Polydimethylsiloxanen, die Alkyl- oder Alkoxygruppen enthalten, die seitenständig sind und/oder am Ende der Siliconkette vorhanden sind, wobei die Gruppen jeweils 2 bis 24 Kohlenstoffatome enthalten;
- Phenylsiliconen, insbesondere Phenyltrimethicon; und
- Kohlenwasserstoffen, die unter geradkettigen und verzweigten, flüchtigen und nichtflüchtigen Kohlenwasserstoffen synthetischer und mineralischer Herkunft ausgewählt werden.

12. Zusammensetzung nach Anspruch 1, wobei die mindestens eine flüssige Fettphase in einer Menge vorhanden ist, die im Bereich von 1 bis 99 Gew.-%, bezogen auf das Gesamtgewicht der Zusammensetzung, liegt.

13. Zusammensetzung nach Anspruch 1, wobei die mindestens eine flüssige Fettphase in einer Menge vorhanden ist, die im Bereich von 10 bis 80 Gew.-%, bezogen auf das Gesamtgewicht der Zusammensetzung, liegt.

14. Zusammensetzung nach Anspruch 1, wobei die flüssige Fettphase mehr als 30 Gew.-%, vorzugsweise mehr als 40 Gew.-% und noch besser 50 bis 100 Gew.-% mindestens eines flüssigen Öls auf Siliconbasis enthält.

15. Zusammensetzung nach Anspruch 1, wobei die mindestens eine flüssige Fettphase mindestens ein flüchtiges Lösemittel enthält, das unter Lösemitteln auf Kohlenwasserstoffbasis und Siliconlösemittel ausgewählt wird, die optional Alkyl- oder Alkoxygruppen umfassen, die seitenständig sind oder am Ende einer Siliconkette vorhanden sind.

16. Zusammensetzung nach Anspruch 1, wobei der Gewichtsanteil des filmbildenden Polymers im Bereich von 2 bis 60 Gew.-%, vorzugsweise 5 bis 60 Gew.-%, noch bevorzugter 2 bis 30 Gew.-% der trockenen Verbindung, bezogen auf das Gesamtgewicht der Zusammensetzung, liegt.

17. Zusammensetzung nach Anspruch 1, die weiterhin mindestens einen zusätzlichen Zusatzstoff enthält, der unter Dispergiermitteln, wie Poly(2-hydroxystearinsäure), Antioxidantien, etherischen Ölen, Konservierungsmitteln, Duftstoffen, Wachsen, Füllstoffen, Neutralisierungsmitteln, kosmetischen und dermatologischen Wirkstoffen, wie zum Beispiel Emollientien, Moisturizer, Vitaminen, essentiellen Fettsäuren, Sonnenschutzmitteln und Gemischen davon ausgewählt wird.

18. Zusammensetzung nach Anspruch 1, wobei das Farbmittel unter lipophilen Farbstoffen, hydrophilen Farbstoffen, Pigmenten und Perlglanzpigmenten ausgewählt wird.

19. Zusammensetzung nach Anspruch 1, wobei das mindestens eine Farbmittel in einem Anteil von 0,01 bis 50 %, vorzugsweise 0,5 bis 40 % und noch bevorzugter 5 bis 30 %, bezogen auf das Gesamtgewicht der Zusammensetzung, vorhanden ist.

20. Zusammensetzung nach Anspruch 1, wobei die Zusammensetzung ein Feststoff ist.

21. Zusammensetzung nach einem der vorhergehenden Ansprüchen,
wobei die Zusammensetzung ein Feststoff ist, der unter den geformten und den gegossenen Stiften ausgewählt wird.

22. Zusammensetzung nach Anspruch 1, wobei die Zusammensetzung in Form eines starren Gels vorliegt.

23. Zusammensetzung nach Anspruch 1, wobei die Zusammensetzung in Form eines wasserfreien Stifts vorliegt.

24. Zusammensetzung nach Anspruch 1, wobei die Zusammensetzung eine wässrige Phase enthält.

25. Zusammensetzung nach Anspruch 1, wobei die Zusammensetzung eine Emulsion ist.

26. Mascara, Eyeliner, Foundation, Lippenstift, Blush, Make-up-Produkt für den Körper, Lidschatten, Gesichtspuder, Concealer-Produkt, der/die/das eine Zusammensetzung umfasst, die mindestens eine flüssige Fettphase in der Mascara, dem Eyeliner, der Foundation, dem Lippenstift, dem Blush, dem Make-up-Produkt für den Körper, dem Lidschatten, dem Gesichtspuder, dem Concealer-Produkt enthält, die eine wie in Anspruch 1 beschriebene Zusammensetzung enthält.

27. Verfahren zum Pflegen, Schminken oder Behandeln von Keratinmaterialien, das das Auftragen einer wie in Anspruch 1 beschriebenen Zusammensetzung auf die Keratinmaterialien umfasst.

## Revendications

1. Composition de maquillage comprenant au moins une phase grasse liquide comprenant
(i) au moins une huile structurée par au moins un polymère polyamide siliconé structurant constitué d'un polymère (homopolymère ou copolymère) ayant une masse moléculaire moyenne en poids allant de 500 à 500 000, contenant au moins un motif de formule (III) 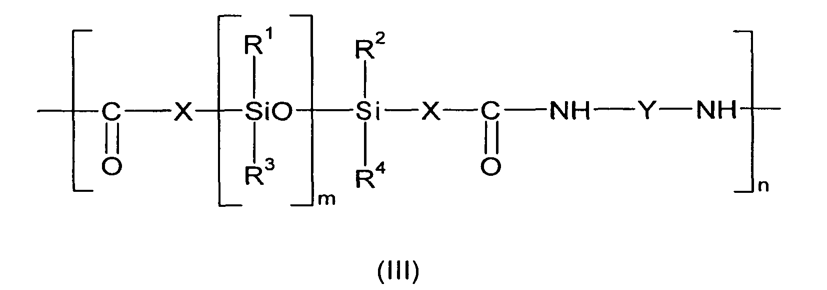 dans laquelle :
1) R¹, R², R³ et R⁴, pouvant être identiques ou différents, représentent un groupement choisi parmi :
les groupements hydrocarbonés, linéaires, ramifiés ou cycliques, en C₁ à C₄₀, saturés ou insaturés, pouvant contenir dans leur chaîne un ou plusieurs atomes d'oxygène, de soufre et/ou d'azote, et pouvant être substitués en partie ou totalement par des atomes de fluor,
les groupements aryles en C₆ à C₁₀, éventuellement substitués par un ou plusieurs groupements alkyle en C₁ à C₄,
les chaînes polyorganosiloxanes contenant éventuellement un ou plusieurs atomes d'oxygène, de soufre et/ou d'azote,
2) les groupements X, pouvant être identiques ou différents, représentent un groupement alkylènediyle, linéaire ou ramifié en C₁ à C₃₀, pouvant contenir dans sa chaîne un ou plusieurs atomes d'oxygène et/ou d'azote,
3) Y est un groupement divalent linéaire ou ramifié alkylène, arylène, cycloalkylène, alkylarylène ou arylalkylène, saturé ou insaturé, en C₁ à C₅₀, pouvant comporter un ou plusieurs atomes d'oxygène, de soufre et/ou d'azote, et/ou porter comme substituant l'un des atomes ou groupes d'atomes suivants:
fluor, hydroxy, cycloalkyle en C₃ à C₈, alkyle en C₁ à C₄₀, aryle en C₅ à C₁₀, phényle éventuellement substitué par 1 à 3 groupements alkyle en C₁ à C₃, hydroxyalkyle en C₁ à C₃ et aminoalkyle en C₁ à C₆, ou
4) Y représente un groupement répondant à la formule : 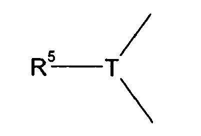 dans laquelle
T représente un groupement hydrocarboné trivalent ou tétravalent, linéaire ou ramifié, saturé ou insaturé, en C₃ à C₂₄ éventuellement substitué par une chaîne polyorganosiloxane, et pouvant contenir un ou plusieurs atomes choisis parmi O, N et S, ou T représente un atome trivalent choisi parmi N, P et Al, et
R⁵ représente un groupement alkyle en C₁ à C₅₀, linéaire ou ramifié, ou une chaîne polyorganosiloxane, pouvant comporter un ou plusieurs groupements ester, amide, uréthane, thiocarbamate, urée, thiourée et/ou sulfonamide qui peut être lié ou non à une autre chaîne du polymère ;
5) n est un entier allant de 2 à 500 et de préférence de 2 à 200, et m est un entier allant de 1 à 1000, de préférence de 1 à 700 et mieux encore de 6 à 200, le polymère étant solide à 25°C et soluble dans ladite huile à une température de 25 à 250°C,
(ii) au moins un polymère filmogène choisi parmi les polymères greffés acrylique-silicone liposolubles, ayant un squelette siliconé et des greffes acryliques ou ayant un squelette acrylique et des greffes siliconées,
ladite huile ayant une affinité pour ledit polymère structurant et éventuellement ledit polymère filmogène, et
la phase grasse liquide, le polymère structurant et le polymère filmogène formant un milieu physiologiquement acceptable,
et
(iii) comprenant en outre au moins un agent colorant.

2. Composition selon la revendication 1, dans laquelle le polymère représente de 0,5% à 80%, de préférence de 2% à 60% et mieux encore de 5% à 40%, par rapport au poids total de la composition.

3. Composition selon la revendication 1, **caractérisée en ce que** ledit au moins un polymère structurant a une température de ramollissement supérieure à 50°C.

4. Composition selon la revendication 1, **caractérisée en ce que** ledit au moins un polymère structurant a une température de ramollissement inférieure à 150°C.

5. Composition selon la revendication 1, **caractérisée en ce que** ledit au moins un polymère structurant a une température de ramollissement allant de 70°C à 130°C.

6. Composition selon la revendication 1, **caractérisée en ce que** ledit au moins un polymère structurant a une masse moléculaire moyenne en poids allant de 500 à 200 000, de préférence allant de 1000 à 100 000, plus préférablement allant de 2000 à 30 000.

7. Composition selon la revendication 1,
**caractérisée en ce que** ladite composition a une dureté allant de 30 à 300 gf.

8. Composition selon la revendication 1, **caractérisée en ce que** ladite composition a une dureté allant de 30 à 250 gf, de préférence de 30 à 200 gf.

9. Composition selon la revendication 1, **caractérisée en ce que** ladite au moins une phase grasse liquide de la composition comprend au moins une huile choisie parmi au moins une huile polaire et au moins une huile apolaire ayant une affinité pour ledit au moins un polymère structurant.

10. Composition selon la revendication 1, **caractérisée en ce que** ladite au moins une huile polaire est choisie parmi:
les huiles végétales hydrocarbonées à forte teneur en triglycérides constitués d'esters d'acides gras et de glycérol dont les acides gras comprennent des chaînes ayant de 4 à 24 atomes de carbone, ces dernières étant éventuellement choisies parmi les chaînes linéaires et ramifiées, et saturées et insaturées ;
- les huiles ou esters de synthèse de formule R₅COOR₆ dans laquelle R₅ est choisi parmi les restes d'acide gras linéaire ou ramifié comportant de 1 à 40 atomes de carbone et R₆ est choisi parmi les chaînes hydrocarbonées contenant de 1 à 40 atomes de carbone, sous réserve que R₅ + R₆ ≥ 10 ;
les éthers de synthèse ayant de 10 à 40 atomes de carbone ;
les alcools gras en C₈ à C₂₆ ;
les acides gras en C₈ à C₂₆.

11. Composition selon la revendication 1, **caractérisée en ce que** ladite au moins une huile apolaire est choisie parmi :
- les huiles de silicone choisies parmi les polydiméthylsiloxanes volatils ou non, linéaires et cycliques, liquides à température ambiante ;
- les polydiméthylsiloxanes comprenant des groupements alkyle ou alcoxy pendants et/ou à l'extrémité de la chaîne siliconée, les groupements contenant chacun de 2 à 24 atomes de carbone ;
- les silicones phénylées, en particulier la phényltriméthicone ; et
- les hydrocarbures choisis parmi les hydrocarbures volatils ou non, linéaires et ramifiés, d'origine synthétique et minérale.

12. Composition selon la revendication 1, **caractérisée en ce que** ladite au moins une phase grasse liquide est présente en une quantité allant de 1% à 99% en poids par rapport au poids total de la composition.

13. Composition selon la revendication 1, **caractérisée en ce que** ladite au moins une phase grasse liquide est présente en une quantité allant de 10% à 80% en poids par rapport au poids total de la composition.

14. Composition selon la revendication 1, dans laquelle la phase grasse liquide contient plus de 30%, de préférence plus de 40% et mieux encore de 50% à 100% en poids d'au moins une huile liquide siliconée.

15. Composition selon la revendication 1,
**caractérisée en ce que** ladite au moins une phase grasse liquide comprend au moins un solvant volatil choisi parmi les solvants hydrocarbonés et les solvants siliconés comprenant éventuellement des groupements alkyle ou alcoxy pendants ou à l'extrémité de la chaîne siliconée.

16. Composition selon la revendication 1, **caractérisée en ce que** la proportion pondérale de polymère filmogène varie de 2-60%, de préférence de 5 à 60%, plus préférablement de 2 à 30% en poids de composé sec par rapport au poids total de la composition.

17. Composition selon la revendication 1, comprenant en outre au moins un additif additionnel choisi parmi les agents dispersants tels que le poly(acide 2-hydroxystéarique), les antioxydants, les huiles essentielles, les conservateurs, les parfums, les cires, les charges, les agents neutralisants, les agents actifs cosmétiques et dermatologiques tels que, par exemple, les émollients, les agents hydratants, les vitamines, les acides gras essentiels, les filtres solaires, et des mélanges de ceux-ci.

18. Composition selon la revendication 1, **caractérisée en ce que** ledit au moins un agent colorant est choisi parmi les colorants lipophiles, les colorants hydrophiles, les pigments et les nacres.

19. Composition selon la revendication 1, **caractérisée en ce que** ledit au moins un agent colorant est présent en une proportion allant de 0,01% à 50%, de préférence de 0,5% à 40% et plus préférablement de 5% à 30% par rapport au poids total de la composition.

20. Composition selon la revendication 1, **caractérisée en ce que** ladite composition est un solide.

21. Composition selon l'une quelconque des revendications précédentes, **caractérisée en ce que** ladite composition est un solide choisi parmi les bâtons moulés et coulés.

22. Composition selon la revendication 1, **caractérisée en ce que** ladite composition est sous forme d'un gel rigide.

23. Composition selon la revendication 1, **caractérisée en ce que** ladite composition est sous forme d'un bâton anhydre.

24. Composition selon la revendication 1, **caractérisée en ce que** ladite composition contient une phase aqueuse.

25. Composition selon la revendication 1, **caractérisée en ce que** ladite composition est une émulsion.

26. Mascara, eye-liner, fond de teint, rouge à lèvres, blush, produit de maquillage du corps, fard à paupières, fard à joues ou produit anticernes comprenant une composition comprenant au moins une phase grasse liquide dans le mascara, l'eye-liner, le fond de teint, le rouge à lèvres, le blush, le produit de maquillage du corps, le fard à paupières, le fard à joues ou le produit anticernes comprenant une composition telle que décrite selon la revendication 1.

27. Procédé de soin, de maquillage ou de traitement de matières kératiniques, comprenant l'application aux matières kératiniques d'une composition telle que décrite selon la revendication 1.
